(19) Europäisches Patentamt
European Patent Office
Office européen des brevets

(11) **EP 2 717 048 B1**

(12) **EUROPEAN PATENT SPECIFICATION**

(45) Date of publication and mention of the grant of the patent:
**01.04.2020 Bulletin 2020/14**

(21) Application number: **12792480.1**

(22) Date of filing: **31.05.2012**

(51) Int Cl.:
***G01N 30/86*** *(2006.01)* ***G01N 30/88*** *(2006.01)*
***G06K 9/00*** *(2006.01)* *G01N 30/74* *(2006.01)*

(86) International application number:
**PCT/JP2012/003612**

(87) International publication number:
**WO 2012/164954 (06.12.2012 Gazette 2012/49)**

(54) **METHOD FOR EVALUATING SIMILARITY OF AGGREGATED DATA, SIMILARITY EVALUATION PROGRAM, AND SIMILARITY EVALUATION DEVICE**

VERFAHREN, PROGRAMM UND VORRICHTUNG ZUR BEURTEILUNG DER ÄHNLICHKEIT AGGREGIERTER DATEN

PROCÉDÉ D'ÉVALUATION DES RESSEMBLANCES ENTRE DONNÉES REGROUPÉES, PROGRAMME ET DISPOSITIF D'ÉVALUATION DES RESSEMBLANCES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.06.2011 JP 2011123847**

(43) Date of publication of application:
**09.04.2014 Bulletin 2014/15**

(73) Proprietor: **TSUMURA & CO.**
**Minato-ku**
**Tokyo 107-8521 (JP)**

(72) Inventors:
- **MORI, Yoshikazu**
  **Inashiki-gun, Ibaraki 300-1192 (JP)**
- **NODA, Keiichi**
  **Inashiki-gun, Ibaraki 300-1192 (JP)**

(74) Representative: **Oppermann, Frank**
**OANDO Oppermann & Oppermann LLP**
**Wilhelminenstrasse 1a**
**65193 Wiesbaden (DE)**

(56) References cited:
**WO-A1-2009/060975** **JP-A- 2005 351 669**
**JP-A- 2007 315 941** **JP-A- 2008 100 918**
**JP-A- 2008 224 235** **US-A1- 2002 028 005**
**US-A1- 2004 034 477**

- **JOHNSON K J ET AL: "High-speed peak matching algorithm for retention time alignment of gas chromatographic data for chemometric analysis", JOURNAL OF CHROMATOGRAPHY, ELSEVIER SCIENCE PUBLISHERS B.V, NL, vol. 996, no. 1-2, 9 May 2003 (2003-05-09) , pages 141-155, XP004427349, ISSN: 0021-9673, DOI: 10.1016/S0021-9673(03)00616-2**

EP 2 717 048 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

FIELD OF THE INVENTION

The present invention relates to a similarity evaluating method, a similarity evaluating program, and a similarity evaluating device for collective data.

DESCRIPTION OF THE PRIOR ART

As multicomponent materials, for example, there are natural product-originated drugs such as kampo medicines that are drugs (hereinafter, referred to as multicomponent drugs) that are composed of multiple components. The quantitative and qualitative profiles of such drugs change due to a geological factor, an ecological factor, collecting season, a collecting area, a collecting aetas, weather during the growing period, and the like of raw material crude drugs.

Thus, for such multicomponent drugs and the like, predetermined criteria are regulated as qualities for securing the safety and the effectiveness thereof, and national supervising agencies, chemical organizations, manufacturing companies, and the like perform quality evaluations based on the criteria.

In general, however, the determination criteria on the quality and the like of a multicomponent drug are set based on the content and the like of one or several distinctive components selected from components in the multicomponent drug.

For example, in Non-Patent Literature 1, in a case where effective components of a multicomponent drug are not identified, it selects a plurality of components that have physical properties such as a quantitatively analyzability, high water-solubility, a undegradability in hot water, and non-chemical reactability with other components and uses the contents of the components acquired through chemical analysis as evaluation criteria.

In addition, it is well known to apply chromatography to a multicomponent drug, obtain an ultraviolet-visible absorption spectrum for each retention time, and set evaluation criteria based on some pieces of component information included therein.

For example, according to Patent Literature 1, some peaks included in HPLC chromatogram data (hereinafter, referred to as a chromatogram) are selected and encoded as barcodes, thereby evaluating a multicomponent drug.

However, in such methods, evaluation targets are limited to "contents of specific components" or "chromatogram peaks of specific components", and thus only some components contained in a multicomponent drug are set as the evaluation targets. Accordingly, since a multicomponent drug includes many components other than components that are evaluation targets, such methods are insufficient as a method of evaluating a multicomponent drug in terms of accuracy.

In order to accurately evaluate the quality of a multicomponent drug, it is necessary to evaluate waveform patterns that cover the all the peak information or almost all peak information with the exclusion of small peaks corresponding to several %. Accordingly, it is necessary to associate all the peaks or almost all peaks with each other between multicomponent drugs.

However, it is difficult to efficiently associate a plurality of peaks with high accuracy. This interferes with an efficient evaluation of multicomponent drugs with high accuracy.

Described more, crude drugs are natural products, and therefore, multicomponent drugs, even which have the same product name, may have slightly different components. Hence, even if drugs have the same quality, content ratios of components thereof may be different from each other or a component present in one drug may not be present in the other drug (hereinafter, referred to as an inter-drug error). In addition, there is also a factor that peak intensity or peak elution time in a chromatogram has no precise reproducibility (hereinafter, referred to as an analysis error). Accordingly, all the peaks of or almost all peaks may not be associated with peaks that are originated from the same components between multicomponent drugs (hereinafter, referred to as peak assignment), thereby interfering with an efficient evaluation with high accuracy.

US 2002/0028005 A1 describes a method for systematically studying proteins to provide data thereon to enable making a catalog of proteins. Proteins are divided into a plurality of spots (peaks) by two-dimensional gel electrophoresis, images of spot patterns (collective date sets) of two gels digitized by an image scanning means are acquired, the spots of the acquired images are superimposed, and similarity (degree of matching) between the spot patterns based on the superimposed spots are detected. Further, the method uses Jaccard coefficient based on the appearing number of peaks when detecting the similarity. US 2002/0028005 A1, however, simply superimposes the images of the spot patterns acquired by the two-dimensional gel electrophoresis and directly matches the corresponding spots with each other one by one as a basis for finding the similarity between the spot patterns.

PRIOR ART LITERATURE

PATENT LITERATURE

PATENT LITERATURE 1: JP 2002-214215 A

NON- PATENT LITERATURE

NON- PATENT LITERATURE1: Pharmaceuticals monthly vol. 28, No. 3, pp 67 to 71 (1986)

SUMMARY OF THE INVENTION

PROBLEM TO BE SOLVED BY THE INVENTION

A problem to be solved is that there is a limit on an efficient evaluation of the quality and the like of multicomponent materials with high accuracy with use of an existing evaluating method.

MEANS FOR SOLVING THE PROBLEM

The problem of the invention is solved with the features of the independent claims. The dependent claims refer to preferred embodiments of the invention.

In order to contribute to improvement of the accuracy and efficiency of the evaluation, the invention provides a similarity evaluating method evaluating similarity between collective data sets in which a plurality of pieces of data are collected (for example, measurement data (chart) such as liquid chromatogram (LC), gas chromatogram (GC) and nuclear magnetic resonance (NMR) spectrum, or data obtained by processing them such as patterning). The method comprises a patterning step of patterning each data of each collective data set with a selected scale, a matching number extraction step of comparing each patterned data in a round-robin to find numbers of matches, and a matching degree determination step of finding a degree of matching with use of Tanimoto coefficient on the basis of the found numbers of matches.

The invention provides a similarity evaluating program to evaluate similarity between collective data sets in which a plurality of pieces of data are collected, the program causing a computer to execute functions. The functions comprises a patterning step of patterning each data of each collective data set with a selected scale, a matching number extraction step of comparing each patterned data in a round-robin to find numbers of matches, and a matching degree determination step of finding a degree of matching with the use of Tanimoto coefficient on the basis of the found numbers of matches.

The invention provides a similarity evaluating device to evaluate similarity between collective data sets in which a plurality of pieces of data are collected, comprising a patterning part that patterns each data of each collective data set with a selected scale, a matching number extraction part that compares each patterned data in a round-robin to find numbers of matches, and a matching degree determination part that finds a degree of matching with the use of Tanimoto coefficient on the basis of the found numbers of matches.

EFFECT OF THE INVENTION

The similarity evaluating method for collective data according to the invention can simply and quickly evaluate similarity between collective data sets, in which a plurality of pieces of data are collected.

Consequently, for example, when comparing a target FP of a multicomponent material of an evaluation target with reference FPs of a plurality of drugs of evaluation criteria to evaluate it, the similarity evaluation method for collective data according to the invention makes it possible to perform simple and quick selection in selecting an FP of a multicomponent material suitable for peak assignment of the target FP from a plurality of reference FPs as a preprocessing thereof.

The similarity evaluating program for collective data according to the invention causes a computer to execute the functions to evaluate similarity between the FPs, thereby performing simple and quick selection of the reference FP and the like.

The similarity evaluating device for collective data according to the invention operates each part to perform simple and quick selection of the reference FP and the like.

BRIEF DESCRIPTION OF THE DRAWINGS

[FIG. 1] It is a block diagram of a similarity evaluating device for collective data (Embodiment 1).
[FIG. 2] It is a process chart of a similarity evaluating method for collective data (Embodiment 1).
[FIG. 3] It is graphs each illustrating a FP of a drug in which (A) is Drug A, (B) is Drug B and (C) is Drug C (Embodiment 1).
[FIG. 4] It is an explanatory diagram illustrating retention time points of a target FP and a reference FP (Embodiment 1).
[FIG. 5] It is an explanatory diagram illustrating a retention time appearance pattern of the target FP (Embodiment 1).
[FIG. 6] It is an explanatory diagram illustrating a retention time appearance pattern of the reference FP (Embodiment 1).
[FIG. 7] It is an explanatory diagram illustrating a number of matches in an appearance distance of the target FP and the reference FP (Embodiment 1).
[FIG. 8] It is an explanatory diagram illustrating numbers of matches for all the retention time appearance distances of the target FP and the reference FP (Embodiment 1).
[FIG. 9] It is an explanatory diagram illustrating degrees of matching for all the retention time appearance patterns of the target FP and the reference FP (Embodiment 1).
[FIG. 10] It is an explanatory diagram illustrating a peak height ratio pattern of the target FP (Second embodiment).
[FIG. 11] It is a flowchart of data processes in the FP similarity evaluating process (Embodiment 1).
[FIG. 12] It is a flowchart of calculation process of the degree of matching between the retention time appearance patterns in the FP similarity evaluating process (Embodiment 1).

EMBODIMENTS FOR CARRYING OUT THE INVENTION

An object being to contribute to improvement of the accuracy and the efficiency of evaluation is accomplished by a patterning part, a matching number extraction part, and a matching degree determination part.

EMBODIMENT 1

Embodiment 1 of the invention applies a similarity evaluating device for collective data, for a preprocessing of an evaluating device for a multicomponent drug that evaluates multicomponent material, for example, a multicomponent drug.
A multicomponent drug is defined as a drug that contains a plurality of effective chemical components, and is not limited thereto, but includes a crude drug, a combination of crude drugs, an extract thereof, kampo medicines and the like. In addition, the dosage form is also not particularly limited, and includes, for example, a liquid, an extract, a capsule, a granule, a pill, a suspension emulsion, a powder, a spirit, a tablet, an infusion decoction, a tincture, a troche, aromatic water, a fluid extract and the like, which are prescribed under "General Rules for Preparations" in the Japanese Pharmacopoeia Fifteenth edition. The multicomponent material also includes those other than the drugs.
Specific examples of the kampo medicines are described in Industry Standard and Voluntarily Revision of "Precautions" in 148 Prescriptions for Medical Kampo Drug Formulation and in Guide to General Kampo Prescription (year 1978).
With the evaluating device for the multicomponent drug, first, it prepares a target FP, which is an extract of unique information from three-dimensional chromatogram data (hereinafter, referred to as 3D Chromatogram) of an evaluation target drug in order to evaluate whether or not the evaluation target drug is equivalent to a plurality of drugs defined as normal products. Next, a FP of a multicomponent drug suitable for peak assignment of the target FP is selected from among a plurality of reference FPs. To the peaks of this selected reference FP, respective peaks of the target FP is assigned.
And, each peak of the target FP assigned as described above is assigned to peak correspondence data of all reference FPs (hereinafter, referred to as the reference group FP) prepared by peak assignment process from all the reference FPs.
Next, the equivalency between peaks of the reference group FPs with the peaks of the assigned target FP (hereinafter, referred to as a target FP assignment peak) is evaluated with MT method. Finally, an acquired evaluation value (hereinafter, referred to as MD value) is compared with a preset determination value (the upper limit value of the MD value), to determine whether the evaluation target drug is equivalent to a normal product or not.
The 3D Chromatogram is a HPLC chromatogram data (hereinafter, referred to as chromatogram) of a kampo medicine as a multicomponent drug that is a multicomponent material as an evaluation target and that includes UV spectra.
An FP is finger print data composed of the maximum values or area values (hereinafter, referred to as peaks) in signal intensity (height) of peaks detected at a specific wavelength and appearance time points (hereinafter, retention time points) of the peaks.

A target FP is acquired by extracting a plurality of peaks, retention time points and UV spectra thereof at a specific detection wavelength from a 3D chromatogram that is three-dimensional chromatogram data of a kampo medicine being an evaluation target. Consequently, the target FP is collective data in which the peaks are collected as a plurality of pieces of data.

A reference FP is an FP of a kampo medicine as a multicomponent drug that is a multicomponent material defined as a normal product, and like the target FP, is acquired by extracting a plurality of peaks, retention time points and UV spectra thereof at a specific detection wavelength from a 3D chromatogram that is three-dimensional chromatogram data. Consequently, the reference FP is also collective data in which peaks are collected as a plurality of pieces of data.

[Similarity Evaluating Device and Similarity Evaluating Method for FPs]

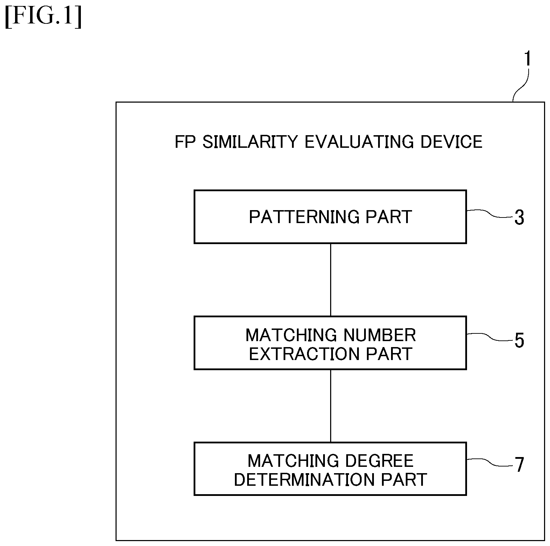

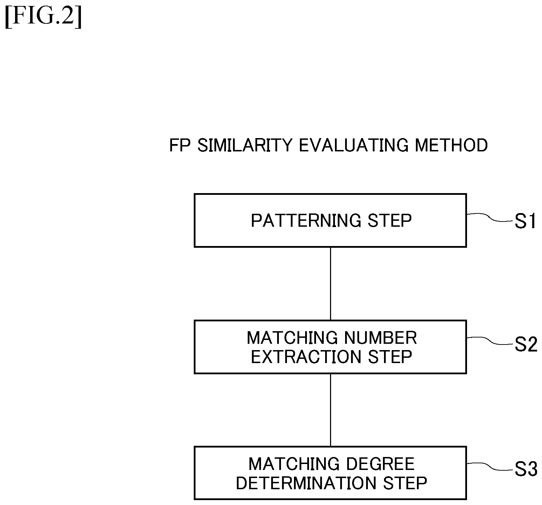

FIG. 1 is a block diagram of the similarity evaluating device for FPs and FIG. 2 is a process chart of the similarity evaluating method for FPs.

As illustrated in FIG. 1 and FIG. 2, the similarity evaluating method of FPs performed by functioning the similarity evaluating device 1 for FPs examines a degree of matching between the target FP and the reference FP.

The similarity evaluating device 1 for FPs is configured by a computer and has CPU, ROM, RAM and the like that are not illustrated. The similarity evaluating device 1 for FPs can implement the similarity evaluating program for collective data installed in a computer, to evaluate similarity of the target FP. However, the smilarity evaluation for the target FP may be realized by using a similarity evaluating program recording medium for collective data that stores the similarity evaluating program thereon by reading out it with the similarity evaluating device 1 for FPs configured by the computer.

The similarity evaluating method for FPs has a patterning step S1 performed by functioning a patterning part 3, a matching number extraction step S2 performed by functioning a matching number extraction part 5, and a matching degree determination step S3 performed by functioning a matching degree determination part 7.

In this similarity evaluating method for FPs, as preprocessing of the final evaluation, a FP of the multicomponent drug suitable for peak assignment of the target FP is selected from among the reference FPs as a plurality of collective data sets.

In the patterning step S1, each peak is each data of the target FP and the reference FP each being a collective data set, and is patterned with a selected scale by the function of the patterning part 3. This scale according to this embodiment is an inter-retention time distance as the appearance distance of the peaks. Specifically, it will be described below.

In the matching number extraction step S2, each patterned peak is compared in a round-robin, to find numbers of matches between respective patterns by the function of the matching number extraction part 5. These numbers of matches is numbers of matches in the appearance distance in this embodiment. Specifically, it will be described below.

In the matching degree determination step S3, a degree of matching between the respective patterns is found on the basis of the found numbers of matches with use of Tanimoto coefficient by the function of the matching degree determination part 7.

In the matching degree determination step S3, the degree of matching is found based on (1 - Tanimoto coefficient) closer to zero in a situation of Tanimoto coefficient being set as:

"a number of matches in appearance distance / (a number of peaks of a target FP + a number of peaks of reference FP – the number of matches in appearance distance)".

This (1 - Tanimoto coefficient) may be weighted by (the number peaks of the target FP - the number of matches in appearance distance + 1) to be converted into "(1 - Tanimoto coefficient) × (the number peaks of the target FP - the number of matches in appearance distance + 1)".

With this weighting, it is possible to select a reference FP having peaks to which the peaks of the target FP matches more. [Operation Principle of Similarity Evaluating Device and Similarity Evaluating Method for FPs]

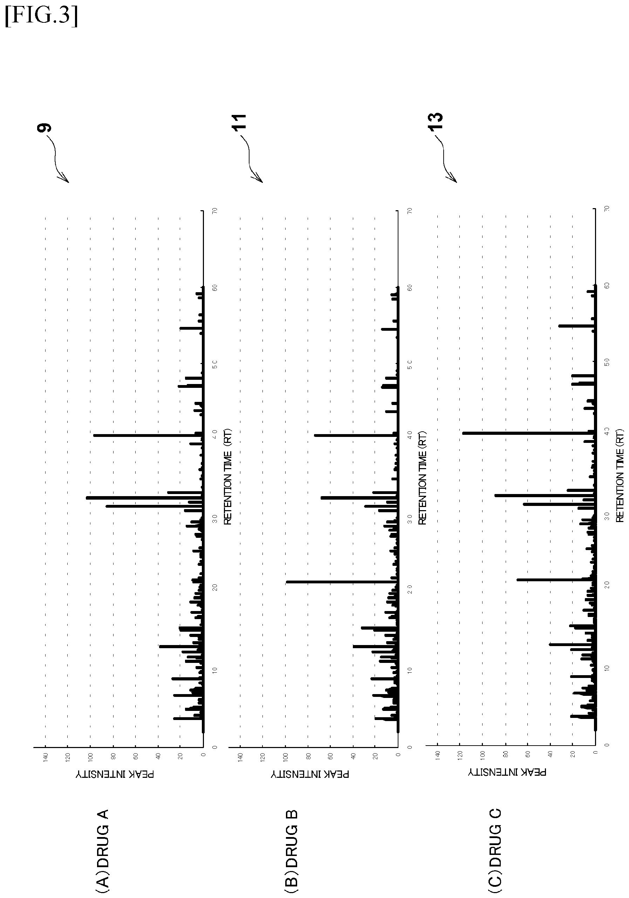

FIG. 3(A) illustrates a FP of Drug A, FIG. 3(B) illustrates a FP of Drug B, and FIG. 3(C) illustrates a FP of Drug C.

For example, if the FP of Drug A is the target FP and the FPs of Drugs B and C are the reference FPs, before each peak of the target FP is assigned to the reference group FPs prepared from Drugs B and C, a reference FP of any one of Drugs B and C suitable for assignment of the target FP is selected from among a plurality of reference FPs, to assign each peak of the target FP to a peak of this selected reference FP.

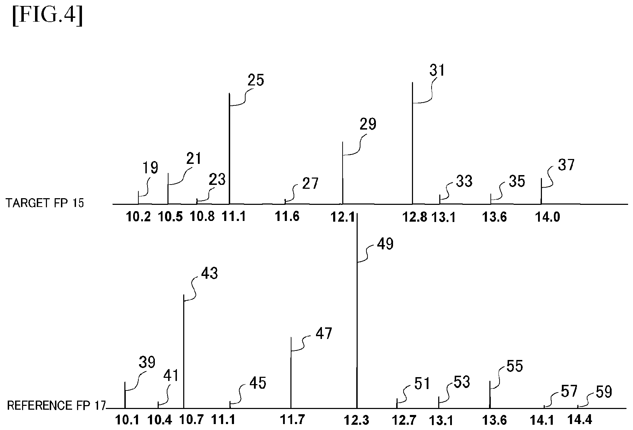

That is, in order to perform the peak assignment of each peak of the target FP with high accuracy, the degrees of matching between the target FP and the reference FPs in the peak retention time appearance pattern are calculated to select a reference FP having the minimum degree of matching from among all the reference FPs as illustrated in FIG. 4 to FIG. 9.

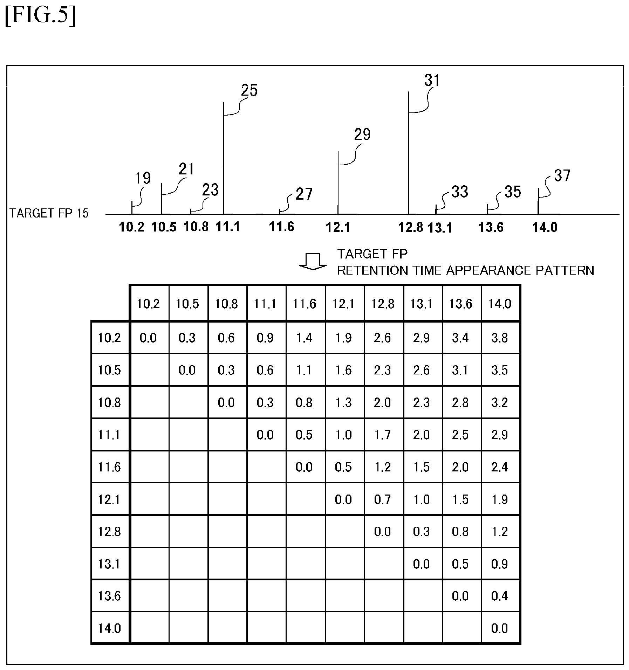

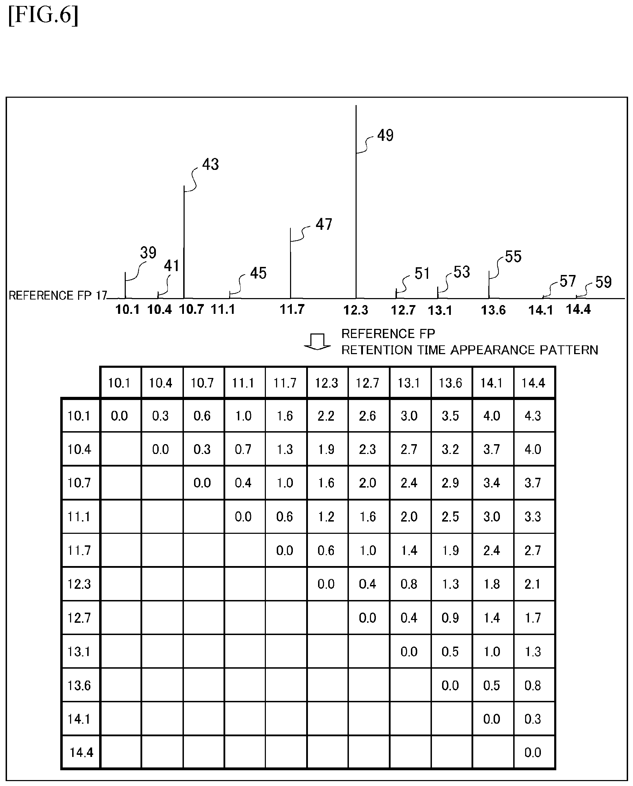

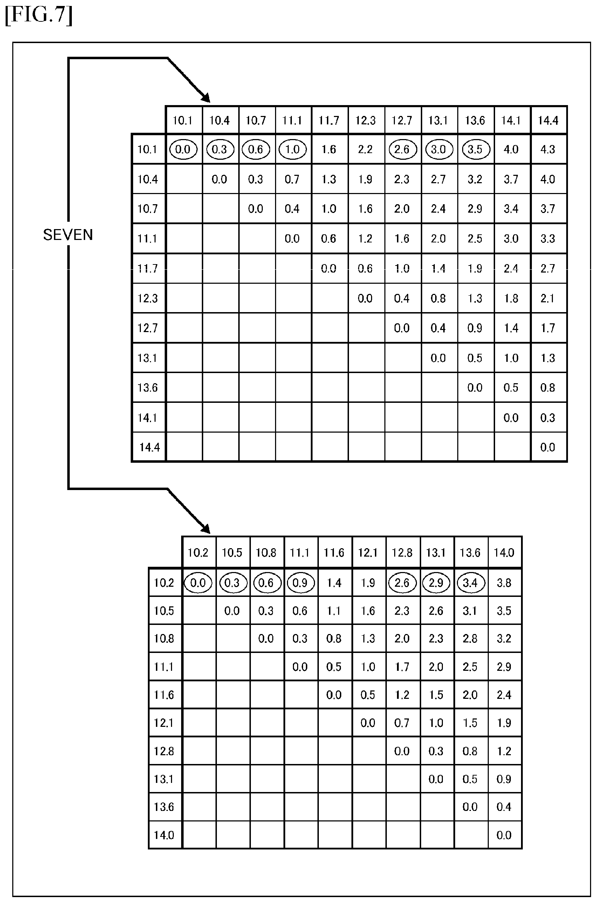

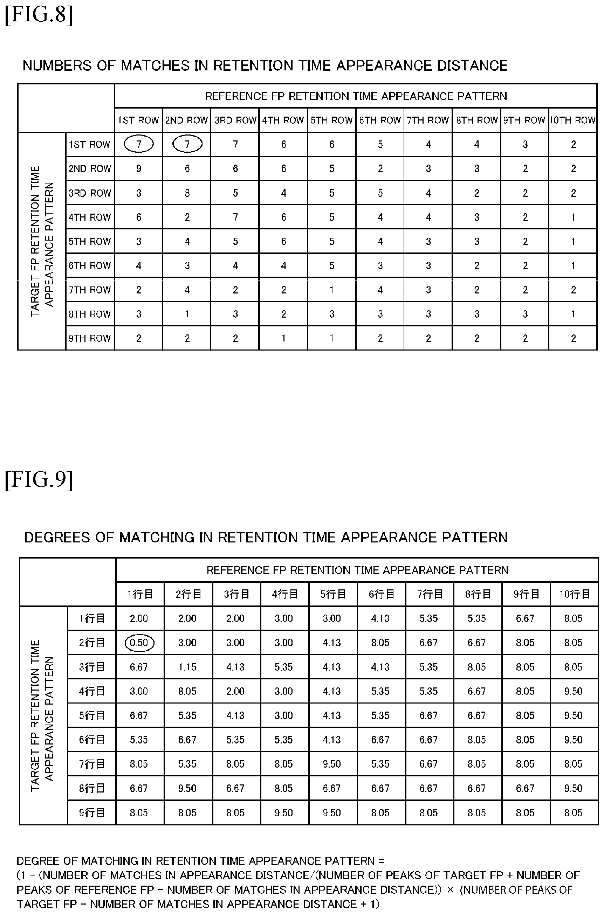

FIG. 4 to FIG. 9 are diagrams that explain the number of matches in the retention time appearance distance or the degree of matching in the retention time appearance pattern between the target FP and the reference FPs. FIG. 4 is an explanatory diagram illustrating the retention time points of the target FP and the reference FP, FIG. 5 is an explanatory diagram illustrating the retention time appearance pattern of the target FP, and FIG. 6 is an explanatory diagram illustrating the retention time appearance pattern of the reference FP. FIG. 7 is an explanatory diagram illustrating the number of matches in the appearance distance between the target and the reference FPs, FIG. 8 is an explanatory diagram illustrating the number of matches of all the retention time appearance distances of the target FP and the reference FP, and FIG. 9 is an explanatory diagram illustrating the degrees of matching of all the retention time appearance patterns of the target FP and the reference FP.

FIG. 4 shows the retention time point of each of the target FP 15 and the reference FP 17. FIG. 5 and FIG. 6 show the retention time appearance patterns in which calculated are all the inter-retention time point distances from each retention time point of the target FP 15 and the reference FP 17 and are summarized in the form of a table. FIG. 7 shows the number of matches in the retention time appearance distance, which is obtained by comparing the values of the retention time appearance patterns of the target FP and the reference FPs in each cell in each row and by counting and calculating a number of which a difference between the two values is within a predetermined range. FIG. 8 shows the numbers of matches in the retention time appearance distance in the form of a table, which are calculated in all combinations of the target FP and the reference FP. FIG. 9 shows the degree of matching between the retention time appearance patterns in the form of a table, which is calculated based on these numbers of matches.

In the peak assignment process of the target FP 15, each peak of the target FP 15 is assigned to a reference FP that is similar to the target FP 15 in the FP pattern as much as possible. It is an important point to select a reference FP that is similar to this target FP 15 from among a plurality of reference FPs in performing the assignment with high accuracy.

Then, as a method of objectively and simply evaluating similarity with respect to the FP pattern of the target FP 15, the similarity in the FP pattern is evaluated according to the degree of matching in the retention time appearance pattern.

For example, in a case where the retention time points of the target FP 15 and the reference FP 17 are illustrated in FIG. 4, the retention time appearance pattern of each of the target FP 15 and the reference FP 17 are as illustrated in FIG. 5 and FIG. 6, respectively. In FIG. 5 and FIG. 6, the target FP 15 and the reference FP 17 in the upper side are patterned and prepared in the form of a table in which value of each cell is an inter-retention time point distance as illustrated in the lower side.

In FIG. 5, the retention time points of respective peaks (19, 21, 23, 25, 27, 29, 31, 33, 35 and 37) of the target FP 15 are (10.2), (10.5), (10.8), (11.1), (11.6), (12.1), (12.8), (13.1), (13.6) and (14.0).

Accordingly, the inter-retention time point distance between the peak 19 and the peak 21 is (10.5) - (10.2) = (0.3). Similarly, the inter-retention time point distance between the peak 19 and the peak 23 is (0.6), and the inter-retention time point distance between the peak 21 and the peak 23 is (0.3), and the like. The followings are similar, and the target FP appearance pattern are acquired as illustrated in the lower side of FIG. 5.

In FIG. 6, the retention time points of respective peaks (39, 41, 43, 45, 47, 49, 51, 53, 55, 57, and 59) of the reference FP 17 are (10.1), (10.4), (10.7), (11.1), (11.7), (12.3), (12.7), (13.1), (13.6), (14.1) and (14.4).

Accordingly, similarly, the inter-retention time point distances are translated into the reference FP appearance pattern as illustrated in the lower side of FIG. 6.

Each peak patterned in FIG. 5 and FIG. 6 is compared in a round-robin to find the numbers of matches. For example, the value of the target FP appearance pattern in each cell of the lower side of FIG. 5 is compared with the value of the reference FP appearance pattern in each cell of the lower side of FIG. 6 as illustrated in FIG. 7, and the numbers of matches are obtained as illustrated in FIG. 8.

In FIG. 7, the patterns according to all the inter-retention time point distance of the retention time appearance patterns of the target FP 15 and the reference FP 17 are compared in a round-robin in sequence on a per-row basis, to calculate the numbers of distances matching within a set range.

For example, when comparing the patterns at the first rows of the target and reference FP retention time appearance patterns of FIG. 7 with each other, circled numerical values match to each other and the number of matches is seven. This matching number of seven is written into a cell for the first rows of the target and reference FP retention time appearance patterns of FIG. 8 as the number of matches in the retention time appearance distance. The same applies to the other rows in FIG. 7, and the 1st to 9th rows of the target FP retention time appearance patterns are compared with the 1st to 10th rows of the reference FP retention time appearance patterns in a round-robin, and the numbers of matches are obtained, respectively.

The results are illustrated in FIG. 8. In FIG. 8, the circled leftmost numerical value of "7" is a result of comparison of the first rows of the respective target and reference FP retention time appearance patterns, and the next numerical value of 7 is a result of comparison of the first row of the target FP retention time appearance pattern with the second row of the reference FP retention time appearance pattern.

In addition, the range of the set value is preferably a range from 0.05 minutes to 0.2 minutes in order to determine the matching of the appearance distances, but is not limited thereto. According to Embodiment 1 , the set value is 0.1 minutes.

When the degree of matching between the retention time appearance patterns is indicated as RP, the degree of matching ($RP_{fg}$) between a retention time appearance pattern at the f-th row of the target FP 15 and a retention time appearance pattern at the g-th row of the reference FP 17 is calculated with use of Tanimoto coefficient as:

$$RP_{fg} = \{1 - (m / (a + b - m))\} \times (a - m + 1).$$

In the equation, "a" is the number of the peaks of the target FP 15 (target FP peak number), "b" is the number of the peaks of the reference FP 17 (reference FP peak number), and "m" is the number of matches in the retention time appearance distance (FIG. 8).

The degrees of matching for each retention time appearance pattern (RP) is calculated by the equation based on the numbers of matches in FIG. 8 (FIG. 9).

RP_min is the minimum value of these RPs and is set as the degree of matching between the retention time appearance patterns of the target FP 15 and the reference FP 17. In FIG. 9, (0.50) is the degree of matching between the target FP 15 and the reference FP.

Such degree of matching is calculated for all the reference FPs, the reference FP having the minimum degree of matching is selected, and the peak assignment of the target FP to this reference FP is performed.

[Similarity Evaluating Program]

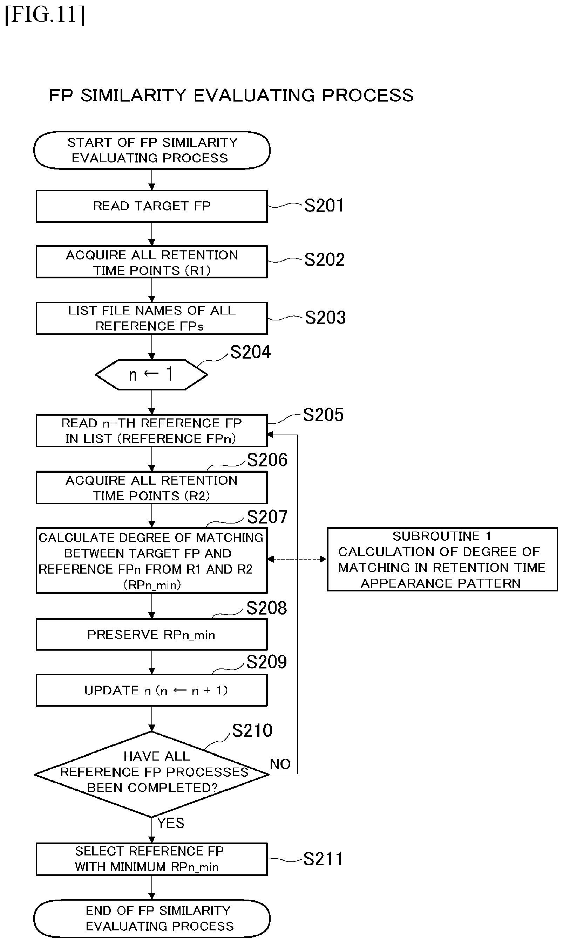

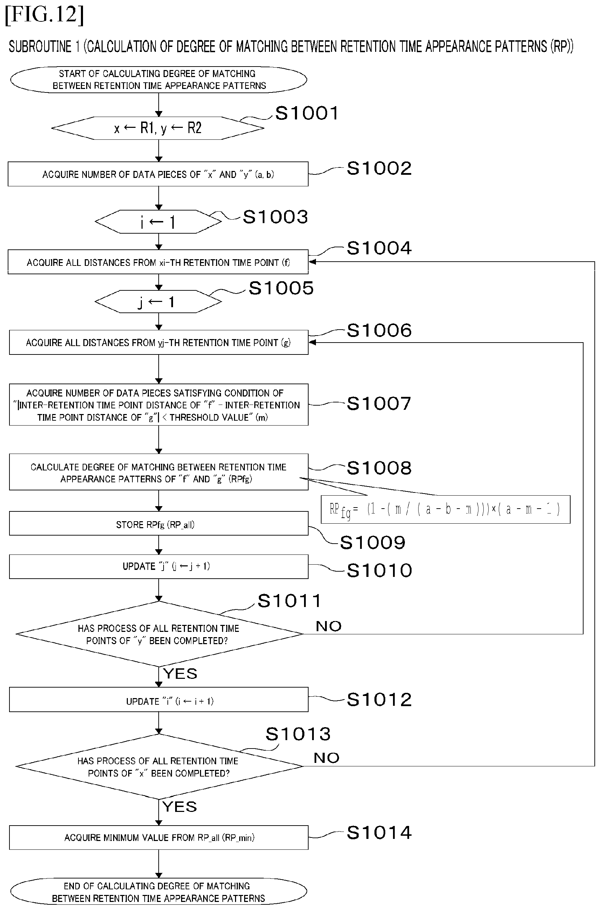

FIGS. 11 and 12 are flowcharts according to the similarity evaluating program.

FIG. 11 is a flowchart illustrating steps of a whole process for evaluating similarity between FPs, wherein the process starts with a system start-up to cause the computer to execute the patterning function, the matching number extraction function, and the matching degree determination function, thereby evaluating the similarity of the retention time appearance patterns between the target FP 17 and a plurality of reference FPs defined as normal products to select a reference FP suitable for assignment of the target FP 17.

FIG. 12 is a flowchart illustrating details of the "Subroutine 1" in the "FP similarity evaluating process" of FIG. 11. This process calculates the degree of matching between the retention time appearance patterns of FPs (for example, the target FP and the reference FP).

[FP Similarity Evaluating Process]

In Step S201, a process of "reading target FP" is executed. This process reads a FP of an assignment target, and the procedure proceeds to Step S202.

In Step S202, a process of "acquiring all retention time points (R1)" is executed. This process acquires all the retention time point information of the target FP read in S201, and the procedure proceeds to Step S203.

In Step S203, a process of "listing file names of all reference FPs" is executed. This process, in order to process all the reference FPs in sequence later, lists file names of all the reference FPs in advance, and the procedure proceeds to Step S204.

In Step S204, as an initial value of a counter for processing the total reference FPs in sequence, "1" is substituted into "n" ($n \leftarrow 1$), and the procedure proceeds to Step S205.

In Step S205, a process of "reading an n-th reference FP in the list (reference $FP_n$)" is executed. At this process, the n-th FP of the file name list of all the reference FPs listed in S203 is read, and the procedure proceeds to Step S206.

In Step S206, a process of "acquiring all retention time points (R2)" is executed. At this process, all the retention time point information of the reference FP read in S205 is acquired totally, and the procedure proceeds to Step S207.

In Step S207, a process of "calculating the degree of matching between retention time appearance patterns of R1 and R2 ($RP_n$_min)" is executed. At this process, $RP_n$_min is calculated from the retention time points of the target FP acquired in S202 and the retention time points of the reference FP acquired in S206, and the procedure proceeds to Step S208. In addition, detailed calculation flows of $RP_n$_min are explained separately by the subroutine 1 in FIG. 12.

In Step S208, a process of "preserving $RP_n$_min ($RP_{all}$_min)" is executed. At this process, $RP_n$_min calculated in S207 is preserved in $RP_{all}$_min, and the procedure proceeds to Step S209.

In Step S209, a process of "updating n (n ← n + 1)" is executed. At this process, "n + 1" is substituted for "n" as the update of "n" to advance the process to the next FP, and the procedure proceeds to Step S210.

In Step S210, a determining process "Have all reference FP processes been completed?" is executed. At this process, it is determined whether all of the reference FPs are processed or not. If processed (YES), the procedure proceeds to Step S211. If there are one or more unprocessed reference FPs (NO), the procedure proceeds to S205 in order to execute the processes of S205 to S210 regarding unprocessed FPs. The processes of S205 to S210 are repeated until the processes of all the reference FPs are completed.

In Step S211, a process of "selecting a reference FP demonstrating the minimum degree of matching from $RP_{all}$_min" is executed. At this process, $RP_1$_min up to $RP_n$_min calculated for all the reference FPs are compared with each other to select a reference FP demonstrating the minimum degree of matching with respect to the retention time appearance pattern of the target FP.

[Subroutine 1]

In Step S1001, a process of "x ← R1, y ← R2" is executed. At this process, R1 and R2 acquired in S202 and S206 of FIG. 11 are respectively substituted into "x" and "y", and the procedure proceeds to Step S1002.

In Step S 1002, a process of "acquiring numbers of data of "x" and "y" (a, b)" is executed. At this process, the numbers of data pieces of "x" and "y" are respectively acquired as "a" and "b", and the procedure proceeds to Step S1003.

In Step S1003, "1" is substituted into "i" (i ← 1) as the initial value of a counter for sequentially invoking the retention time points of "x", and the procedure proceeds to Step S1004.

In Step S1004, a process of "acquiring all distances from the xi-th retention time point (f)" is performed. In this process, all distances, from the xi-th retention time point, of retention time points after the xi-th retention time point are acquired as "f', and the procedure proceeds to Step S1005.

In Step S1005, "1" is substituted into "j" (j ← 1) as the initial value of a counter for sequentially invoking the retention time points of "y", and the procedure proceeds to Step S1006.

In Step S1006, a process of "acquiring all distances from the yj-th retention time point (g)" is performed. In this process, all distances, from the yj-th retention time point, of retention time points after the yj-th retention time point are acquired as "g", and the procedure proceeds to Step S1007.

In Step S1007, a process of "acquiring the number of data pieces satisfying a relation of "|inter-retention time point distance of "f" - inter-retention time point distance of "g"| < threshold value" (m)" is performed. In this process, an inter-retention time point distances "f" and "g" acquired in Steps S1004 and S1006 are compared with each other in a round-robin, the number of data pieces satisfying the condition of "|inter-retention time point distance of "f" - inter-retention time point distance of "g"| < threshold value" is acquired as "m", and the procedure proceeds to Step S1008.

In Step S1008, a process of "calculating the degree of matching between the retention time appearance patterns of "f' and "g" ($RP_{fg}$)" is performed. In this process, $RP_{fg}$ is calculated based on "a" and "b" acquired in Step S1002 and "m" acquired in Step S1007 as:

$$RP_{fg} = (1 - (m/(a + b - m))) \times (a - m + 1).$$

Then, the procedure proceeds to Step S1009.

In Step S1009, a process of "preserving $RP_{fg}$ (RP_all)" is executed. At this process, the degree of matching calculated in S1008 is preserved to RP_all, and the procedure proceeds to Step S1010.

In Step S1010, a process of "updating "j" (j ← j + 1)" is executed. At this process, "j + 1" is substituted into "j" as the update of "j" in order to advance the process of "y" to the next retention time point, and the procedure proceeds to Step S1011.

In Step S1011, a determining process "Has the process been completed at all the retention time points of "y"?" is executed. In this process, it is determined whether or not the process for all the retention time points of "y" has been completed. If completed (YES), it is determined that the process for all the retention time points has been completed to proceed to Step S1012. If not completed (NO), it is determined that one or more retention time points that have not been processed remain in "y", to proceed to Step S1006. In other words, the processes of Steps S1006 to S1011 are repeated until all the retention time points of "y" are processed.

In Step S1012, a process of "updating "i" (i ← i + 1)" is executed. At this process, "i + 1" is substituted into "i" as the update of "i" in order to advance the process of "x" to the next retention time point, and the procedure proceeds to Step S1013.

In Step S1013, a determining process "Has the process been completed at all the retention time points of "x"?" is executed. In this process, it is determined whether or not the process for all the retention time points of "x" has been completed. If completed (YES), it is determined that the process for all the retention time points of "x" has been completed to proceed to Step S1014. If not completed (NO), it is determined that one ore more retention time points that have not been processed remain in "x", to proceed to Step S1004. In other words, the processes of Steps S1004 to S1013 are repeated until all the retention time points of "x" are processed.
In Step S1014, a process of "acquiring a minimum value from RP_all (RP_min)" is performed. In this process, the minimum value in RP_all in which RPs for all the combinations of the retention time appearance patterns of the target FP and the reference FP are stored is acquired as RP_min, and RP_min is input to Step S207 of Fig. 11 to finish the process of calculating the degree of matching between the retention time appearance patterns.

[Effects of Embodiment 1]

Embodiment 1 of the invention is the similarity evaluating method for a FP to evaluate similarity between the target FP 15 and the reference FP 17, in which a plurality of peaks (19, 21, ...) and (39, 41, ...) are collected. The method includes the patterning step S1 of patterning each of the peaks (19, 21, ...) and (39, 41, ...) of the target FP 15 and the reference FP 17 with the appearance distance as illustrated in FIGS. 5 and 6, the matching number extraction step S2 of comparing each patterned pattern in a round-robin to find the numbers of matches as illustrated in FIG. 8, and the matching degree determination step S3 of finding the degree of matching as illustrated in FIG. 9 with the use of Tanimoto coefficient on the basis of the found numbers of matches.
Consequently, it is possible to evaluate similarity between the target FP 15 and the reference FP 17 simply and quickly, thereby assigning each peak of the target FP 15 to the reference FP that is similar to the target FP 15 in the FP pattern as much as possible. It is possible to select a reference FP that is similar to this target FP 15 from a plurality of reference FPs, thereby performing the assignment with higher accuracy.
In the matching degree determination step S3, the Tanimoto coefficient is set as "a number of matches in an appearance distance/(a number of peaks of a target FP + a number of peaks of a reference FP - the number of matches in the appearance distance)" to find the degree of matching with (1 - Tanimoto coefficient) closer to zero. The (1 - Tanimoto coefficient) is weighted by (the number of peaks of the target FP - the number of matches in the appearance distance + 1) to be converted into "(1 - Tanimoto coefficient) × (the number of peaks of the target FP - the number of matches in the appearance distance + 1)", thereby finding the degree of matching.
Accordingly, it is possible to select a reference FP that matches more to the peaks (19, 21, ...) of the target FP 15 due to the weighting.
The similarity evaluation program for collective data according to Embodiment 1 of the present invention can evaluate similarity between FPs by executing the patterning function, the matching number extraction function, and the matching degree determination function, thereby simply and quickly performing selection of the reference FP.
According to the similarity evaluating device 1 of FPs of Embodiment 1 of the invention, it is possible to realize the similarity evaluating method for FPs by the patterning part 3, the matching number extraction part 5, and the matching degree determination part 7.

EMBODIMENT 2

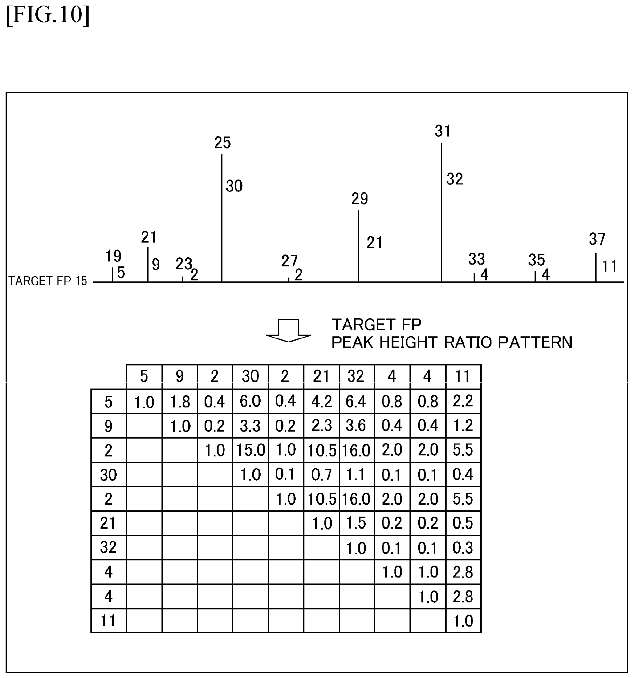

FIG. 10 is an explanatory diagram illustrating a peak height ratio pattern of a target FP.
In Embodiment 2, the target FP 15 in the upper side of FIG. 10 is patterned in the form of a table in which value of each cell is a peak height ratio as illustrated in the lower side.
In FIG. 10, the peak heights of respective peaks (19, 21, 23, 25, 27, 29, 31, 33, 35, 37) of the target FP 15 are (5, 9, 2, 30, 2, 21, 32, 4, 4, 11).
Therefore, the height ratio between the peak 19 and the peak 21 is (9 ÷ 5) = (1.8). Similarly, the height ratio between the peak 19 and the peak 23 is (0.4), the height ratio between the peak 21 and the peak 23 is (0.2), and the like. The followings are similar, and the height ratio pattern of the target FP is acquired as illustrated in the lower side of FIG. 10.
Also for the reference FPs, the height ratio patterns of the peaks of the reference FPs are acquired similarly.
Therefore, in Embodiment 2, the patterning step S1 performs patterning with the height ratio for the peaks as a scale.
In the matching number extraction step S2, the number of matches in the height ratio is set as the matching number, and each patterned peak with the height ratio of the peak is compared in a round-robin, to calculate the number of the height ratio matching within a set range. From this calculation, it is possible to obtain the matching number similarly to FIG. 8.
In addition, this embodiment of patterning with the height ratio of the peak may have a plurality of identical values in a single row illustrated in the lower side of FIG. 10 and there is a need not to count these values a plurality of times.

The matching degree determination step S2 sets the Tanimoto coefficient as "a number of matches in height ratio/(a number of peaks of a target FP + a number of peaks of a reference FP - the number of matches in the height ratio)" to find the degree of matching with (1 - Tanimoto coefficient) closer to zero.

Further, the (1 - Tanimoto coefficient) is weighted by (the number of peaks of the target FP - the number of matches in the height ratio + 1) to be converted into "(1 - Tanimoto coefficient) × (the number of peaks of the target FP - the number of matches in the height ratio + 1)", to select a reference FP that matches more to the peaks (19, 21, ...) of the target FP 15 due to the weighting.

Therefore, Embodiment 2 can provide similar effects to those of Embodiment 1.

[Others]

Although the embodiments of the present invention are applied to evaluation of the kampo medicine as a multicomponent drug, the present invention may be applied to evaluation of other multicomponent materials. The chromatogram is not limited to the 3D chromatogram, and a FP may be used as what composed of peaks with the exclusion of UV spectra and of retention time points thereof.

The similarity evaluation method for collective data according to the present invention is a similarity evaluating method to examine the degree of matching between collective data sets in which a plurality of pieces of data are collected. The similarity evaluation method includes a patterning step of patterning each data of each collective data set with a selected scale, a matching number extraction step of comparing each patterned data in a round-robin to find numbers of matches, and a matching degree determination step of finding a degree of matching with use of Tanimoto coefficient on the basis of the found numbers of matches, thereby being broadly applied to evaluation of similarity between collective data sets. The collective data set is not limited to a FP, and may be also applied to other signal data and the like.

The FP as the collective data set of the aforementioned embodiments is prepared on the basis of the peak heights, to be evaluated similarity by the aforementioned method. However, even when a FP is prepared with area values of peaks, the FP can be evaluated in the same way.

That is, peaks used in the similarity evaluating method, the similarity evaluating program and the similarity evaluating device for collective data according to the present invention encompasses a case where a peak means the maximum value of signal intensity (height) as described above and also a case where a peak means an area value of signal intensity (peak area) is expressed as the height.

In this case, even when a FP is prepared with the peak areas, the FP is prepared by expressing the peak areas as heights. As the FP, it is a similar expression to the case where the FP is prepared with the peak heights of the aforementioned embodiment. Consequently, even if the FP is prepared with the peak areas, it is possible to evaluate similarity by the process of the aforementioned Embodiment 1 or Embodiment 2 in the same way as a case where the FP is prepared with the peak heights of signal intensity.

Therefore, in the invention, the patterning part, the patterning step, and the patterning function can process in the same way as Embodiment 2 with use of area ratio of the peak area as a scale in which each data of each collective data set is selected other than the peak appearance distance of Embodiment 1.

DESCRIPTION OF THE NUMERALS

1 Similarity evaluating device for FPs (Similarity evaluating device for collective data)
3 Patterning part
5 Matching number extraction part
7 Matching degree determination part
S1 Patterning step
S2 Matching number extraction step
S3 Matching degree determination step

## Claims

1. A similarity evaluating method for collective data to evaluate similarity between collective data sets in which a plurality of pieces of data are collected, the collective data sets being finger print data (FP) (15, 17) composed of maximum values or area values in signal intensity of peaks (19, 21, 23, 25, 27, 29, 31, 33, 35, 37; 39, 41, 43, 45, 47, 49, 51, 53, 55, 57, 59) and retention time points of said peaks, the method comprising:

a patterning step (S1) of patterning each peak of each collective data set with a selected scale;
a matching number extraction step (S2) of comparing each patterned peak in a round-robin to find numbers of matches; and
a matching degree determination step (S3) of finding a degree of matching with use of Tanimoto coefficient on the basis of the found numbers of matches, **characterized in that**
the patterning step (S1) takes any one of appearance distance, height ratio and area ratio of a peak as the scale, and acquires appearance distances, height ratios and area ratios between each peak and all subsequent peaks of the collective data sets as patterns according to the scale, and
the matching number extraction step (S2) compares the acquired appearance distances, height ratios or area ratios with each other in a round-robin between the collective data sets, to find numbers of matches between the patterns in the appearance distance, height ratio or area ratio according to the scale.

**2.** The method according to claim 1, **characterized in that**
in a case where a reference finger print data (FP) (17) being most similar to a target finger print data (FP) (15) is selected from plural kinds of reference finger print data according to the degree of matching,
the matching degree determination step sets the Tanimoto coefficient as "a number of matches in any one of appearance distance, height ratio and area ratio/(a number of peaks of a target FP + a number of peaks of a reference FP - the number of matches in any one of appearance distance, height ratio and area ratio)" to find the degree of matching with (1 - Tanimoto coefficient) closer to zero.

**3.** The method according to claim 2, **characterized in that**
the (1 - Tanimoto coefficient) is weighted by (the number of peaks of target FP - the number of matches in any one of appearance distance, height ratio and area ratio + 1) to be converted into "(1 - Tanimoto coefficient) × (the number of peaks of target FP - the number of matches in any one of appearance distance, height ratio and area ratio + 1)".

**4.** The method according to claim 2 or 3, **characterized in that**
the finger print data (FP) is detected from a chromatogram of a multicomponent material.

**5.** The method according to claim 4, **characterized in that**
the multicomponent material is a multicomponent drug that is one of a crude drug, a combination of crude drugs, an extract thereof, and a kampo medicine.

**6.** A similarity evaluating program for collective data to evaluate similarity between collective data sets in which a plurality of pieces of data are collected, the collective data sets being finger print data (FP) (15, 17) composed of maximum values or area values in signal intensity of peaks (19, 21, 23, 25, 27, 29, 31, 33, 35, 37; 39, 41, 43, 45, 47, 49, 51, 53, 55, 57, 59) and retention time points of said peaks, the program causing a computer to execute functions comprising:

a patterning function of patterning each peak of each collective data set with a selected scale;
a matching number extraction function of comparing each patterned peak in a round-robin to find numbers of matches; and
a matching degree determination function of finding a degree of matching with use of Tanimoto coefficient on the basis of the found numbers of matches, **characterized in that**
the patterning function takes any one of appearance distance, height ratio and area ratio of a peak as the scale, and acquires appearance distances, height ratios and area ratios between each peak and all subsequent peaks of the collective data sets as patterns according to the scale, and
the matching number extraction function compares the acquired appearance distances, height ratios or area ratios with each other in a round-robin between the collective data sets, to find numbers of matches between the patterns in the appearance distance, height ratio or area ratio according to the scale.

**7.** The program according to claim 6, **characterized in that**
in a case where a reference finger print data (FP) (17) being most similar to a target finger print data (FP) (15) is selected from plural kinds of reference finger print data according to the degree of matching,
the matching degree determination function sets the Tanimoto coefficient as "a number of matches in any one of appearance distance, height ratio and area ratio/(a number of peaks of a target FP + a number of peaks of a reference FP - the number of matches in any one of appearance distance, height ratio and area ratio)" to find the degree of matching with (1 - Tanimoto coefficient) closer to zero.

8. The program according to claim 7, **characterized in that** the (1 - Tanimoto coefficient) is weighted by (the number of peaks of target FP - the number of matches in any one of appearance distance, height ratio and area ratio + 1) to be converted into "(1 - Tanimoto coefficient) × (the number of peaks of target FP - the number of matches in any one of appearance distance, height ratio and area ratio + 1)".

9. The program according to claim 7 or 8, **characterized in that** the finger print data (FP) is detected from a chromatogram of a multicomponent material.

10. The program according to claim 9, **characterized in that** the multicomponent material is a multicomponent drug that is one of a crude drug, a combination of crude drugs, an extract thereof, and a kampo medicine.

11. A similarity evaluating device to evaluate similarity between collective data sets in which a plurality of pieces of data are collected, the collective data sets being finger print data (FP) (15, 17) composed of maximum values or area values in signal intensity of peaks (19, 21, 23,25, 27, 29, 31, 33, 35, 37; 39, 41, 43, 45, 47, 49, 51, 53, 55, 57, 59) and retention time points of said peaks, the device comprising:

a patterning part (3) of patterning each peak of each collective data set with a selected scale;
a matching number extraction part (5) of comparing each patterned peak in a round-robin to find numbers of matches; and
a matching degree determination part (7) of finding a degree of matching with use of Tanimoto coefficient on the basis of the found numbers of matches, **characterized in that**
the patterning part (3) takes any one of appearance distance, height ratio and area ratio of a peak as the scale, and acquires appearance distances, height ratios and area ratios between each peak and all subsequent peaks of the collective data sets as patterns according to the scale, and
the matching number extraction part (7) compares the acquired appearance distances, height ratios or area ratios with each other in a round-robin between the collective data sets, to find numbers of matches between the patterns in the appearance distance, height ratio or area ratio according to the scale.

12. The device according to claim 11, **characterized in that** in a case where a reference finger print data (FP) (17) being most similar to a target finger print data (FP) (15) is selected from plural kinds of reference finger print data (FP) according to the degree of matching,
the matching degree determination part (7) sets the Tanimoto coefficient as "a number of matches in any one of appearance distance, height ratio and area ratio/(a number of peaks of a target FP + a number of peaks of a reference FP - the number of matches in any one of appearance distance, height ratio and area ratio)" to find the degree of matching with (1 - Tanimoto coefficient) closer to zero.

13. The device according to claim 12, **characterized in that** the (1 - Tanimoto coefficient) is weighted by (the number of peaks of target FP - the number of matches in any one of appearance distance, height ratio and area ratio + 1) to be converted into "(1 - Tanimoto coefficient) × (the number of peaks of target FP - the number of matches in any one of appearance distance, height ratio and area ratio + 1)".

14. The device according to claim 12 or 13, **characterized in that** the finger print data (FP) is detected from a chromatogram of a multicomponent material.

15. The device according to claim 14, **characterized in that** the multicomponent material is a multicomponent drug that is one of a crude drug, a combination of crude drugs, an extract thereof, and a kampo medicine.

## Patentansprüche

1. Ein Verfahren zur Beurteilung der Ähnlichkeit für gesammelte Daten zur Beurteilung der Ähnlichkeit von erfassten Datensätzen, die eine Mehrzahl von Daten erfassen, wobei die erfassten Datensätze Fingerabdruck-Daten (FP) (15, 17) sind, die sich aus Maximalwerten oder Flächenwerten in der Signalstärke von Peaks (19, 21, 23, 25, 27, 29, 31, 33, 35, 37; 39, 41, 43, 45, 47, 49, 51, 53, 55, 57, 59) und Retentionszeit-Punkten von diesen Peaks zusammensetzen, wobei das Verfahren aufweist:

einen Strukturierungsschritt (S1) des Strukturierens jedes Peaks von jedem erfassten Datensatz mit einer ausgewählten Skalierung;
einen Übereinstimmungsanzahl-Extrahierungsschritt (S2) des Vergleichens jedes strukturierten Peaks in einem Rundlauf-Verfahren (Round Robin) zum Auffinden der Anzahl der Übereinstimmungen; und
einen Übereinstimmungsgrad-Bestimmungsschritt (S3) des Auffindens eines Übereinstimmungsgrads mittels eines Tanimoto-Koeffizienten auf der Grundlage der aufgefundenen Anzahl von Übereinstimmungen, **dadurch gekennzeichnet, dass**
der Strukturierungsschritt (S1) irgendeinen von dem Erscheinungsabstand, Höhenverhältnis und Flächenverhältnis eines Peaks als die Skalierung nimmt, und die Erscheinungsabstände, Höhenverhältnisse und Flächenverhältnisse zwischen jedem Peak und sämtlichen nachfolgenden Peaks der erfassten Datensätze als Muster entsprechend der Skalierung ermittelt, und
der Übereinstimmungsanzahl-Extrahierungsschritt (S2) die ermittelten Erscheinungsabstände, Höhenverhältnisse oder Flächenverhältnisse miteinander in einem Rundlauf-Verfahren (Round Robin) zwischen den erfassten Datensätzen vergleicht, um die Anzahl von Übereinstimmungen zwischen den Mustern in dem Erscheinungsabstand, Höhenverhältnis oder Flächenverhältnis entsprechend der Skalierung aufzufinden.

**2.** Das Verfahren nach Anspruch 1, **dadurch gekennzeichnet, dass**
in dem Fall, in dem Referenz-Fingerabdruck-Daten (FP) (17), welche mit Ziel-Fingerabdruck-Daten (FP) (15) am meisten übereinstimmen, aus den verschiedenen Arten von Referenz-Fingerabdruck-Daten entsprechend dem Übereinstimmungsgrad ausgewählt werden,
der Übereinstimmungsgrad-Bestimmungsschritt den Tanimoto-Koeffizienten als "eine Anzahl von Übereinstimmungen in irgendeinem von dem Erscheinungsabstand, Höhenverhältnis und Flächenverhältnis/(eine Anzahl von Peaks von einem Ziel-FP + eine Anzahl von Peaks von einem Referenz-FP - die Anzahl von Überreinstimmungen in irgendeinem von dem Erscheinungsabstand, Höhenverhältnis und Flächenverhältnis)" setzt, um den Übereinstimmungsgrad mit (1- Tanimoto-Koeffizient) näher zu Null aufzufinden.

**3.** Das Verfahren nach Anspruch 2, **dadurch gekennzeichnet, dass**
der (1- Tanimoto-Koeffizient) gewichtet wird durch (die Anzahl von Peaks von einem Ziel-FP - die Anzahl von Überreinstimmungen in irgendeinem von dem Erscheinungsabstand, Höhenverhältnis und Flächenverhältnis + 1), um in "(1- Tanimoto-Koeffizient) x (die Anzahl von Peaks des Ziel-FP - die Anzahl von Übereinstimmungen in irgendeinem von dem Erscheinungsabstand, Höhenverhältnis und Flächenverhältnis + 1)" konvertiert zu werden.

**4.** Das Verfahren nach Anspruch 2 oder 3, **dadurch gekennzeichnet, dass** die Fingerabdruckdaten (FP) von einem Chromatogramm eines Multikomponentenmaterials detektiert werden.

**5.** Das Verfahren nach Anspruch 4, **dadurch gekennzeichnet, dass** das Multikomponentenmaterial ein Multikomponentenmedikament ist, das ein Crude-Medikament oder ein Extrakt davon oder ein Kampo-Medikament ist.

**6.** Ein Programm zur Beurteilung der Ähnlichkeit für erfasste Daten zur Beurteilung der Ähnlichkeit von erfassten Datensätzen, die eine Mehrzahl von Daten erfassen, wobei die erfassten Datensätze Fingerabdruck-Daten (FP) (15, 17) sind, die sich aus Maximalwerten oder Flächenwerten in der Signalstärke von Peaks (19, 21, 23, 25, 27, 29, 31, 33, 35, 37; 39, 41, 43, 45, 47, 49, 51, 53, 55, 57, 59) und Retentionszeit-Punkten von diesen Peaks zusammensetzen, wobei das Programm einen Computer dazu veranlasst die folgenden Funktionen auszuführen:

eine Strukturierungsfunktion des Strukturierens jedes Peaks von jedem erfassten Datensatz mit einer ausgewählten Skalierung;
eine Übereinstimmungsanzahl-Extrahierungsfunktion des Vergleichens jedes strukturierten Peaks in einem Rundlauf-Verfahren (Round Robin) zum Auffinden der Anzahl der Übereinstimmungen; und
eine Übereinstimmungsgrad-Bestimmungsfunktion des Auffindens eines Übereinstimmungsgrads mittels eines Tanimoto-Koeffizienten auf der Grundlage der aufgefundenen Anzahl von Übereinstimmungen, **dadurch gekennzeichnet, dass**
die Strukturierungsfunktion irgendeinen von dem Erscheinungsabstand, Höhenverhältnis und Flächenverhältnis eines Peaks als die Skalierung nimmt, und die Erscheinungsabstände, Höhenverhältnisse und Flächenverhältnisse zwischen jedem Peak und sämtlichen nachfolgenden Peaks der erfassten Datensätze als Muster entsprechend der Skalierung ermittelt, und
die Übereinstimmungsanzahl-Extrahierungsfunktion die ermittelten Erscheinungsabstände, Höhenverhältnisse oder Flächenverhältnisse miteinander in einem Rundlauf-Verfahren (Round Robin) zwischen den erfassten Datensätzen vergleicht, um die Anzahl von Übereinstimmungen zwischen den Mustern in dem Erscheinungsabstand, Höhenverhältnis oder Flächenverhältnis entsprechend der Skalierung aufzufinden.

7. Das Programm nach Anspruch 6, **dadurch gekennzeichnet, dass**
in dem Fall, in dem Referenz-Fingerabdruck-Daten (FP) (17), welche mit Ziel-Fingerabdruck-Daten (FP) (15) am meisten übereinstimmen, aus den verschiedenen Arten von Referenz-Fingerabdruck-Daten entsprechend dem Übereinstimmungsgrad ausgewählt werden,
die Übereinstimmungsgrad-Bestimmungsfunktion den Tanimoto-Koeffizienten als "eine Anzahl von Übereinstimmungen in irgendeinem von dem Erscheinungsabstand, Höhenverhältnis und Flächenverhältnis/(eine Anzahl von Peaks von einem Ziel-FP + eine Anzahl von Peaks von einem Referenz-FP - die Anzahl von Überreinstimmungen in irgendeinem von dem Erscheinungsabstand, Höhenverhältnis und Flächenverhältnis)" setzt, um den Übereinstimmungsgrad mit (1- Tanimoto-Koeffizient) näher zu Null aufzufinden.

8. Das Programm nach Anspruch 7, **dadurch gekennzeichnet, dass**
der (1- Tanimoto-Koeffizient) gewichtet wird durch (die Anzahl von Peaks von einem Ziel-FP - die Anzahl von Überreinstimmungen in irgendeinem von dem Erscheinungsabstand, Höhenverhältnis und Flächenverhältnis + 1), um in "(1- Tanimoto-Koeffizient) x (die Anzahl von Peaks des Ziel-FP - die Anzahl von Übereinstimmungen in irgendeinem von dem Erscheinungsabstand, Höhenverhältnis und Flächenverhältnis + 1)" konvertiert zu werden.

9. Das Programm nach Anspruch 7 oder 8, **dadurch gekennzeichnet, dass** die Fingerabdruckdaten (FP) von einem Chromatogramm eines Multikomponentenmaterials detektiert werden.

10. Das Programm nach Anspruch 9, **dadurch gekennzeichnet, dass** das Multikomponentenmaterial ein Multikomponentenmedikament ist, das ein Crude-Medikament oder ein Extrakt davon oder ein Kampo-Medikament ist.

11. Eine Vorrichtung zur Beurteilung der Ähnlichkeit zum Beurteilen der Ähnlichkeit von erfassten Datensätzen, die eine Mehrzahl von Daten erfassen, wobei die erfassten Datensätze Fingerabdruck-Daten (FP) (15, 17) sind, die sich aus Maximalwerten oder Flächenwerten in der Signalstärke von Peaks (19, 21, 23, 25, 27, 29, 31, 33, 35, 37; 39, 41, 43, 45, 47, 49, 51, 53, 55, 57, 59) und Retentionszeit-Punkten von diesen Peaks zusammensetzen, wobei die Vorrichtung aufweist:

ein Strukturierungsteil (3) des Strukturierens jedes Peaks von jedem erfassten Datensatz mit einer ausgewählten Skalierung;
ein Übereinstimmungsanzahl-Extrahierungsteil (5) des Vergleichens jedes strukturierten Peaks in einem Rundlauf-Verfahren (Round Robin) zum Auffinden der Anzahl der Übereinstimmungen; und
ein Übereinstimmungsgrad-Bestimmungsteil (7) des Auffindens eines Übereinstimmungsgrads mittels eines Tanimoto-Koeffizienten auf der Grundlage der aufgefundenen Anzahl von Übereinstimmungen, **dadurch gekennzeichnet, dass**
das Strukturierungsteil (3) irgendeinen von dem Erscheinungsabstand, Höhenverhältnis und Flächenverhältnis eines Peaks als die Skalierung nimmt, und die Erscheinungsabstände, Höhenverhältnisse und Flächenverhältnisse zwischen jedem Peak und sämtlichen nachfolgenden Peaks der erfassten Datensätze als Muster entsprechend der Skalierung ermittelt, und
das Übereinstimmungsanzahl-Extrahierungsteil (7) die ermittelten Erscheinungsabstände, Höhenverhältnisse oder Flächenverhältnisse miteinander in einem Rundlauf-Verfahren (Round Robin) zwischen den erfassten Datensätzen vergleicht, um die Anzahl von Übereinstimmungen zwischen den Mustern in dem Erscheinungsabstand, Höhenverhältnis oder Flächenverhältnis entsprechend der Skalierung aufzufinden.

12. Die Vorrichtung nach Anspruch 11, **dadurch gekennzeichnet, dass**
in dem Fall, in dem Referenz-Fingerabdruck-Daten (FP) (17), welche mit Ziel-Fingerabdruck-Daten (FP) (15) am meisten übereinstimmen, aus den verschiedenen Arten von Referenz-Fingerabdruck-Daten entsprechend dem Übereinstimmungsgrad ausgewählt werden,
das Übereinstimmungsgrad-Bestimmungsteil (7) den Tanimoto-Koeffizienten als "eine Anzahl von Übereinstimmungen in irgendeinem von dem Erscheinungsabstand, Höhenverhältnis und Flächenverhältnis/(eine Anzahl von Peaks von einem Ziel-FP + eine Anzahl von Peaks von einem Referenz-FP - die Anzahl von Überreinstimmungen in irgendeinem von dem Erscheinungsabstand, Höhenverhältnis und Flächenverhältnis)" setzt, um den Übereinstimmungsgrad mit (1- Tanimoto-Koeffizient) näher zu Null aufzufinden.

13. Die Vorrichtung nach Anspruch 12, **dadurch gekennzeichnet, dass**
der (1- Tanimoto-Koeffizient) gewichtet wird durch (die Anzahl von Peaks von einem Ziel-FP - die Anzahl von Überreinstimmungen in irgendeinem von dem Erscheinungsabstand, Höhenverhältnis und Flächenverhältnis + 1), um in "(1- Tanimoto-Koeffizient) x (die Anzahl von Peaks des Ziel-FP - die Anzahl von Übereinstimmungen in irgendeinem von dem Erscheinungsabstand, Höhenverhältnis und Flächenverhältnis + 1)" konvertiert zu werden.

**14.** Die Vorrichtung nach Anspruch 12 oder 13, **dadurch gekennzeichnet, dass** die Fingerabdruckdaten (FP) von einem Chromatogramm eines Multikomponentenmaterials detektiert werden.

**15.** Die Vorrichtung nach Anspruch 14, **dadurch gekennzeichnet, dass** das Multikomponentenmaterial ein Multikomponentenmedikament ist, das ein Crude-Medikament oder ein Extrakt davon oder ein Kampo-Medikament ist.

## Revendications

**1.** Procédé d'évaluation de similitude de données collectives permettant d'évaluer une similitude entre des jeux de données collectives dans lesquels une pluralité de données sont recueillies, les jeux de données collectives étant des données d'empreintes digitales (FP) (15, 17) composées de valeurs maximales ou de valeurs d'aire d'intensité de signal de crêtes (19, 21, 23, 25, 27, 29 31, 33, 35, 37 ; 39, 41, 43, 45, 47, 49, 51, 53, 55, 57, 59) et de points de temps de rétention desdites crêtes, le procédé comprenant :

une étape de modélisation (S1) destinée à la modélisation de chaque crête de chaque jeu de données collectives avec une échelle sélectionnée ;
une étape d'extraction de nombre de concordances (S2) destinée à la comparaison de chaque crête modélisée à tour de rôle pour découvrir des nombres de concordances ; et
une étape de détermination de degré de concordance (S3) destinée à la découverte d'un degré de concordance en utilisant un coefficient de Tanimoto sur la base des nombres de concordances découverts, **caractérisé en ce que** l'étape de modélisation (S1) prend l'un quelconque d'une distance d'apparence, d'un rapport de hauteur et d'un rapport d'aire d'une crête en tant que l'échelle, et acquiert des distances d'apparence, des rapports de hauteur et des rapports d'aire entre chaque crête et toutes les crêtes suivantes des jeux de données collectives en tant que modèles en fonction de l'échelle, et
l'étape d'extraction de nombre de concordances (S2) compare les distances d'apparence, les rapports de hauteur ou les rapports d'aire acquis les uns aux autres à tour de rôle entre les jeux de données collectives, pour découvrir des nombres de concordances entre les modèles de la distance d'apparence, du rapport de hauteur ou du rapport d'aire en fonction de l'échelle.

**2.** Procédé selon la revendication 1, **caractérisé en ce que**
dans un cas où des données d'empreintes digitales (FP) de référence (17) qui sont les plus similaires à des données d'empreintes digitales (FP) cibles (15) sont sélectionnées parmi plusieurs types de données d'empreintes digitales de référence en fonction du degré de concordance,
l'étape de détermination de degré de concordance définit le coefficient de Tanimoto en tant qu'« un nombre de concordances de l'un quelconque d'une distance d'apparence, d'un rapport de hauteur et d'un rapport d'aire/(un nombre de crêtes d'une FP cible + un nombre de crêtes d'une FP de référence - le nombre de concordances de l'un quelconque d'une distance d'apparence, d'un rapport de hauteur et d'un rapport d'aire) » pour découvrir le degré de concordance avec (1 - coefficient de Tanimoto) plus proche de zéro.

**3.** Procédé selon la revendication 2, **caractérisé en ce que**
le (1 - coefficient de Tanimoto) est pondéré par (le nombre de crêtes de FP cible - le nombre de concordances de l'un quelconque d'une distance d'apparence, d'un rapport de hauteur et d'un rapport d'aire + 1) à convertir en « (1 - coefficient de Tanimoto) x (le nombre de crêtes de FP cible - le nombre de concordances de l'un quelconque d'une distance d'apparence, d'un rapport de hauteur et d'un rapport d'aire + 1) ».

**4.** Procédé selon la revendication 2 ou 3, **caractérisé en ce que**
les données d'empreintes digitales (FP) sont détectées à partir d'un chromatogramme d'un matériau à multiples composants.

**5.** Procédé selon la revendication 4, **caractérisé en ce que**
le matériau à multiples composants est un médicament à multiples composants qui est l'un d'un médicament brut, d'une combinaison de médicaments bruts, d'un extrait de celui-ci, et d'un médicament Kampo.

6. Programme d'évaluation de similitude de données collectives permettant d'évaluer une similitude entre des jeux de données collectives dans lesquels une pluralité de données sont recueillies, les jeux de données collectives étant des données d'empreintes digitales (FP) (15, 17) composées de valeurs maximales ou de valeurs d'aire d'intensité de signal de crêtes (19, 21, 23, 25, 27, 29 31, 33, 35, 37 ; 39, 41, 43, 45, 47, 49, 51, 53, 55, 57, 59) et de points de temps de rétention desdites crêtes, le programme amenant un ordinateur à exécuter des fonctions comprenant :

   une fonction de modélisation destinée à la modélisation de chaque crête de chaque jeu de données collectives avec une échelle sélectionnée ;
   une fonction d'extraction de nombre de concordances destinée à la comparaison de chaque crête modélisée à tour de rôle pour découvrir des nombres de concordances ; et
   une fonction de détermination de degré de concordance destinée à la découverte d'un degré de concordance en utilisant un coefficient de Tanimoto sur la base des nombres de concordances découverts, **caractérisé en ce que**
   la fonction de modélisation prend l'un quelconque d'une distance d'apparence, d'un rapport de hauteur et d'un rapport d'aire d'une crête en tant que l'échelle, et acquiert des distances d'apparence, des rapports de hauteur et des rapports d'aire entre chaque crête et toutes les crêtes suivantes des jeux de données collectives en tant que modèles en fonction de l'échelle, et
   la fonction d'extraction de nombre de concordances compare les distances d'apparence, les rapports de hauteur ou les rapports d'aire acquis les uns aux autres à tour de rôle entre les jeux de données collectives, pour découvrir des nombres de concordances entre les modèles de la distance d'apparence, du rapport de hauteur ou du rapport d'aire en fonction de l'échelle.

7. Programme selon la revendication 6, **caractérisé en ce que**
dans un cas où des données d'empreintes digitales (FP) de référence (17) qui sont les plus similaires à des données d'empreintes digitales (FP) cibles (15) sont sélectionnées parmi plusieurs types de données d'empreintes digitales de référence en fonction du degré de concordance,
la fonction de détermination de degré de concordance définit le coefficient de Tanimoto en tant qu'« un nombre de concordances de l'un quelconque d'une distance d'apparence, d'un rapport de hauteur et d'un rapport d'aire/(un nombre de crêtes d'une FP cible + un nombre de crêtes d'une FP de référence - le nombre de concordances de l'un quelconque d'une distance d'apparence, d'un rapport de hauteur et d'un rapport d'aire) » pour découvrir le degré de concordance avec (1 - coefficient de Tanimoto) plus proche de zéro.

8. Programme selon la revendication 7, **caractérisé en ce que** le (1 - coefficient de Tanimoto) est pondéré par (le nombre de crêtes de FP cible - le nombre de concordances de l'un quelconque d'une distance d'apparence, d'un rapport de hauteur et d'un rapport d'aire + 1) à convertir en « (1 - coefficient de Tanimoto) x (le nombre de crêtes de FP cible - le nombre de concordances de l'un quelconque d'une distance d'apparence, d'un rapport de hauteur et d'un rapport d'aire + 1) ».

9. Programme selon la revendication 7 ou 8, **caractérisé en ce que** les données d'empreintes digitales (FP) sont détectées à partir d'un chromatogramme d'un matériau à multiples composants.

10. Programme selon la revendication 9, **caractérisé en ce que** le matériau à multiples composants est un médicament à multiples composants qui est l'un d'un médicament brut, d'une combinaison de médicaments bruts, d'un extrait de celui-ci, et d'un médicament Kampo.

11. Dispositif d'évaluation de similitude permettant d'évaluer une similitude entre des jeux de données collectives dans lesquels une pluralité de données sont recueillies, les jeux de données collectives étant des données d'empreintes digitales (FP) (15, 17) composées de valeurs maximales ou de valeurs d'aire d'intensité de signal de crêtes (19, 21, 23, 25, 27, 29 31, 33, 35, 37 ; 39, 41, 43, 45, 47, 49, 51, 53, 55, 57, 59) et de points de temps de rétention desdites crêtes, le dispositif comprenant :

   une partie de modélisation (3) destinée à la modélisation de chaque crête de chaque jeu de données collectives avec une échelle sélectionnée ;
   une partie d'extraction de nombre de concordances (5) destinée à la comparaison de chaque crête modélisée à tour de rôle pour découvrir des nombres de concordances ; et
   une partie de détermination de degré de concordance (7) destinée à la découverte d'un degré de concordance en utilisant un coefficient de Tanimoto sur la base des nombres de concordances découverts, **caractérisé en ce que** la partie de modélisation (3) prend l'un quelconque d'une distance d'apparence, d'un rapport de hauteur et d'un rapport d'aire d'une crête en tant que l'échelle, et acquiert des distances d'apparence, des rapports de hauteur et des rapports d'aire entre chaque crête et toutes les crêtes suivantes des jeux de données collectives en tant que modèles en fonction de l'échelle, et
la partie d'extraction de nombre de concordances (7) compare les distances d'apparence, les rapports de hauteur ou les rapports d'aire acquis les uns aux autres à tour de rôle entre les jeux de données collectives, pour découvrir des nombres de concordances entre les modèles de la distance d'apparence, du rapport de hauteur ou du rapport d'aire en fonction de l'échelle.

**12.** Dispositif selon la revendication 11, **caractérisé en ce que**
dans un cas où des données d'empreintes digitales (FP) de référence (17) qui sont les plus similaires à des données d'empreintes digitales (FP) cibles (15) sont sélectionnées parmi plusieurs types de données d'empreintes digitales (FP) de référence en fonction du degré de concordance,
la partie de détermination de degré de concordance (7) définit le coefficient de Tanimoto en tant qu'« un nombre de concordances de l'un quelconque d'une distance d'apparence, d'un rapport de hauteur et d'un rapport d'aire/(un nombre de crêtes d'une FP cible + un nombre de crêtes d'une FP de référence - le nombre de concordances de l'un quelconque d'une distance d'apparence, d'un rapport de hauteur et d'un rapport d'aire) » pour découvrir le degré de concordance avec (1 - coefficient de Tanimoto) plus proche de zéro.

**13.** Dispositif selon la revendication 12, **caractérisé en ce que** le (1 - coefficient de Tanimoto) est pondéré par (le nombre de crêtes de FP cible - le nombre de concordances de l'un quelconque d'une distance d'apparence, d'un rapport de hauteur et d'un rapport d'aire + 1) à convertir en « (1 - coefficient de Tanimoto) x (le nombre de crêtes de FP cible - le nombre de concordances de l'un quelconque d'une distance d'apparence, d'un rapport de hauteur et d'un rapport d'aire + 1) ».

**14.** Dispositif selon la revendication 12 ou 13, **caractérisé en ce que** les données d'empreintes digitales (FP) sont détectées à partir d'un chromatogramme d'un matériau à multiples composants.

**15.** Dispositif selon la revendication 14, **caractérisé en ce que** le matériau à multiples composants est un médicament à multiples composants qui est l'un d'un médicament brut, d'une combinaison de médicaments bruts, d'un extrait de celui-ci, et d'un médicament Kampo.

[FIG.1]

1
FP SIMILARITY EVALUATING DEVICE
PATTERNING PART
3
MATCHING NUMBER EXTRACTION PART
5
MATCHING DEGREE DETERMINATION PART
7

[FIG.2]

FP SIMILARITY EVALUATING METHOD
PATTERNING STEP
S1
MATCHING NUMBER EXTRACTION STEP
S2
MATCHING DEGREE DETERMINATION STEP
S3

[FIG.3]

9
11
13
(A) DRUG A
(B) DRUG B
(C) DRUG C
PEAK INTENSITY
RETENTION TIME (RT)

[FIG.4]

TARGET FP 15
19
21
23
25
27
29
31
33
35
37
10.2 10.5 10.8 11.1 11.6 12.1 12.8 13.1 13.6 14.0
REFERENCE FP 17
39
41
43
45
47
49
51
53
55
57
59
10.1 10.4 10.7 11.1 11.7 12.3 12.7 13.1 13.6 14.1 14.4

[FIG.5]

TARGET FP 15
19
21
23
25
27
29
31
33
35
37
10.2 10.5 10.8 11.1 11.6 12.1 12.8 13.1 13.6 14.0
TARGET FP
RETENTION TIME APPEARANCE PATTERN

| | 10.2 | 10.5 | 10.8 | 11.1 | 11.6 | 12.1 | 12.8 | 13.1 | 13.6 | 14.0 |
|---|---|---|---|---|---|---|---|---|---|---|
| 10.2 | 0.0 | 0.3 | 0.6 | 0.9 | 1.4 | 1.9 | 2.6 | 2.9 | 3.4 | 3.8 |
| 10.5 | | 0.0 | 0.3 | 0.6 | 1.1 | 1.6 | 2.3 | 2.6 | 3.1 | 3.5 |
| 10.8 | | | 0.0 | 0.3 | 0.8 | 1.3 | 2.0 | 2.3 | 2.8 | 3.2 |
| 11.1 | | | | 0.0 | 0.5 | 1.0 | 1.7 | 2.0 | 2.5 | 2.9 |
| 11.6 | | | | | 0.0 | 0.5 | 1.2 | 1.5 | 2.0 | 2.4 |
| 12.1 | | | | | | 0.0 | 0.7 | 1.0 | 1.5 | 1.9 |
| 12.8 | | | | | | | 0.0 | 0.3 | 0.8 | 1.2 |
| 13.1 | | | | | | | | 0.0 | 0.5 | 0.9 |
| 13.6 | | | | | | | | | 0.0 | 0.4 |
| 14.0 | | | | | | | | | | 0.0 |

[FIG.6]

49
43
47
39
41
45
51
53
55
57
59
REFERENCE FP 17
10.1 10.4 10.7 11.1 11.7 12.3 12.7 13.1 13.6 14.1 14.4
REFERENCE FP
RETENTION TIME APPEARANCE PATTERN

| | 10.1 | 10.4 | 10.7 | 11.1 | 11.7 | 12.3 | 12.7 | 13.1 | 13.6 | 14.1 | 14.4 |
|---|---|---|---|---|---|---|---|---|---|---|---|
| 10.1 | 0.0 | 0.3 | 0.6 | 1.0 | 1.6 | 2.2 | 2.6 | 3.0 | 3.5 | 4.0 | 4.3 |
| 10.4 | | 0.0 | 0.3 | 0.7 | 1.3 | 1.9 | 2.3 | 2.7 | 3.2 | 3.7 | 4.0 |
| 10.7 | | | 0.0 | 0.4 | 1.0 | 1.6 | 2.0 | 2.4 | 2.9 | 3.4 | 3.7 |
| 11.1 | | | | 0.0 | 0.6 | 1.2 | 1.6 | 2.0 | 2.5 | 3.0 | 3.3 |
| 11.7 | | | | | 0.0 | 0.6 | 1.0 | 1.4 | 1.9 | 2.4 | 2.7 |
| 12.3 | | | | | | 0.0 | 0.4 | 0.8 | 1.3 | 1.8 | 2.1 |
| 12.7 | | | | | | | 0.0 | 0.4 | 0.9 | 1.4 | 1.7 |
| 13.1 | | | | | | | | 0.0 | 0.5 | 1.0 | 1.3 |
| 13.6 | | | | | | | | | 0.0 | 0.5 | 0.8 |
| 14.1 | | | | | | | | | | 0.0 | 0.3 |
| 14.4 | | | | | | | | | | | 0.0 |

[FIG.7]

SEVEN

| | 10.1 | 10.4 | 10.7 | 11.1 | 11.7 | 12.3 | 12.7 | 13.1 | 13.6 | 14.1 | 14.4 |
|---|---|---|---|---|---|---|---|---|---|---|---|
| 10.1 | 0.0 | 0.3 | 0.6 | 1.0 | 1.6 | 2.2 | 2.6 | 3.0 | 3.5 | 4.0 | 4.3 |
| 10.4 | | 0.0 | 0.3 | 0.7 | 1.3 | 1.9 | 2.3 | 2.7 | 3.2 | 3.7 | 4.0 |
| 10.7 | | | 0.0 | 0.4 | 1.0 | 1.6 | 2.0 | 2.4 | 2.9 | 3.4 | 3.7 |
| 11.1 | | | | 0.0 | 0.6 | 1.2 | 1.6 | 2.0 | 2.5 | 3.0 | 3.3 |
| 11.7 | | | | | 0.0 | 0.6 | 1.0 | 1.4 | 1.9 | 2.4 | 2.7 |
| 12.3 | | | | | | 0.0 | 0.4 | 0.8 | 1.3 | 1.8 | 2.1 |
| 12.7 | | | | | | | 0.0 | 0.4 | 0.9 | 1.4 | 1.7 |
| 13.1 | | | | | | | | 0.0 | 0.5 | 1.0 | 1.3 |
| 13.6 | | | | | | | | | 0.0 | 0.5 | 0.8 |
| 14.1 | | | | | | | | | | 0.0 | 0.3 |
| 14.4 | | | | | | | | | | | 0.0 |

| | 10.2 | 10.5 | 10.8 | 11.1 | 11.6 | 12.1 | 12.8 | 13.1 | 13.6 | 14.0 |
|---|---|---|---|---|---|---|---|---|---|---|
| 10.2 | 0.0 | 0.3 | 0.6 | 0.9 | 1.4 | 1.9 | 2.6 | 2.9 | 3.4 | 3.8 |
| 10.5 | | 0.0 | 0.3 | 0.6 | 1.1 | 1.6 | 2.3 | 2.6 | 3.1 | 3.5 |
| 10.8 | | | 0.0 | 0.3 | 0.8 | 1.3 | 2.0 | 2.3 | 2.8 | 3.2 |
| 11.1 | | | | 0.0 | 0.5 | 1.0 | 1.7 | 2.0 | 2.5 | 2.9 |
| 11.6 | | | | | 0.0 | 0.5 | 1.2 | 1.5 | 2.0 | 2.4 |
| 12.1 | | | | | | 0.0 | 0.7 | 1.0 | 1.5 | 1.9 |
| 12.8 | | | | | | | 0.0 | 0.3 | 0.8 | 1.2 |
| 13.1 | | | | | | | | 0.0 | 0.5 | 0.9 |
| 13.6 | | | | | | | | | 0.0 | 0.4 |
| 14.0 | | | | | | | | | | 0.0 |

[FIG.8]

NUMBERS OF MATCHES IN RETENTION TIME APPEARANCE DISTANCE

| | | REFERENCE FP RETENTION TIME APPEARANCE PATTERN | | | | | | | | | |
|---|---|---|---|---|---|---|---|---|---|---|---|
| | | 1ST ROW | 2ND ROW | 3RD ROW | 4TH ROW | 5TH ROW | 6TH ROW | 7TH ROW | 8TH ROW | 9TH ROW | 10TH ROW |
| TARGET FP RETENTION TIME APPEARANCE PATTERN | 1ST ROW | 7 | 7 | 7 | 6 | 6 | 5 | 4 | 4 | 3 | 2 |
| | 2ND ROW | 9 | 6 | 6 | 6 | 5 | 2 | 3 | 3 | 2 | 2 |
| | 3RD ROW | 3 | 8 | 5 | 4 | 5 | 5 | 4 | 2 | 2 | 2 |
| | 4TH ROW | 6 | 2 | 7 | 6 | 5 | 4 | 4 | 3 | 2 | 1 |
| | 5TH ROW | 3 | 4 | 5 | 6 | 5 | 4 | 3 | 3 | 2 | 1 |
| | 6TH ROW | 4 | 3 | 4 | 4 | 5 | 3 | 3 | 2 | 2 | 1 |
| | 7TH ROW | 2 | 4 | 2 | 2 | 1 | 4 | 3 | 2 | 2 | 2 |
| | 8TH ROW | 3 | 1 | 3 | 2 | 3 | 3 | 3 | 3 | 3 | 1 |
| | 9TH ROW | 2 | 2 | 2 | 1 | 1 | 2 | 2 | 2 | 2 | 2 |

[FIG.9]

DEGREES OF MATCHING IN RETENTION TIME APPEARANCE PATTERN

| | | REFERENCE FP RETENTION TIME APPEARANCE PATTERN | | | | | | | | | |
|---|---|---|---|---|---|---|---|---|---|---|---|
| | | 1行目 | 2行目 | 3行目 | 4行目 | 5行目 | 6行目 | 7行目 | 8行目 | 9行目 | 10行目 |
| TARGET FP RETENTION TIME APPEARANCE PATTERN | 1行目 | 2.00 | 2.00 | 2.00 | 3.00 | 3.00 | 4.13 | 5.35 | 5.35 | 6.67 | 8.05 |
| | 2行目 | 0.50 | 3.00 | 3.00 | 3.00 | 4.13 | 8.05 | 6.67 | 6.67 | 8.05 | 8.05 |
| | 3行目 | 6.67 | 1.15 | 4.13 | 5.35 | 4.13 | 4.13 | 5.35 | 8.05 | 8.05 | 8.05 |
| | 4行目 | 3.00 | 8.05 | 2.00 | 3.00 | 4.13 | 5.35 | 5.35 | 6.67 | 8.05 | 9.50 |
| | 5行目 | 6.67 | 5.35 | 4.13 | 3.00 | 4.13 | 5.35 | 6.67 | 6.67 | 8.05 | 9.50 |
| | 6行目 | 5.35 | 6.67 | 5.35 | 5.35 | 4.13 | 6.67 | 6.67 | 8.05 | 8.05 | 9.50 |
| | 7行目 | 8.05 | 5.35 | 8.05 | 8.05 | 9.50 | 5.35 | 6.67 | 8.05 | 8.05 | 8.05 |
| | 8行目 | 6.67 | 9.50 | 6.67 | 8.05 | 6.67 | 6.67 | 6.67 | 6.67 | 6.67 | 9.50 |
| | 9行目 | 8.05 | 8.05 | 8.05 | 9.50 | 9.50 | 8.05 | 8.05 | 8.05 | 8.05 | 8.05 |

DEGREE OF MATCHING IN RETENTION TIME APPEARANCE PATTERN =
(1 − (NUMBER OF MATCHES IN APPEARANCE DISTANCE/(NUMBER OF PEAKS OF TARGET FP + NUMBER OF PEAKS OF REFERENCE FP − NUMBER OF MATCHES IN APPEARANCE DISTANCE)) × (NUMBER OF PEAKS OF TARGET FP − NUMBER OF MATCHES IN APPEARANCE DISTANCE + 1)

[FIG.10]

TARGET FP 15
19
5
21
9
23
2
25
30
27
2
29
21
31
32
33
4
35
4
37
11
TARGET FP
PEAK HEIGHT RATIO PATTERN


| | 5 | 9 | 2 | 30 | 2 | 21 | 32 | 4 | 4 | 11 |
|---|---|---|---|---|---|---|---|---|---|---|
| 5 | 1.0 | 1.8 | 0.4 | 6.0 | 0.4 | 4.2 | 6.4 | 0.8 | 0.8 | 2.2 |
| 9 | | 1.0 | 0.2 | 3.3 | 0.2 | 2.3 | 3.6 | 0.4 | 0.4 | 1.2 |
| 2 | | | 1.0 | 15.0 | 1.0 | 10.5 | 16.0 | 2.0 | 2.0 | 5.5 |
| 30 | | | | 1.0 | 0.1 | 0.7 | 1.1 | 0.1 | 0.1 | 0.4 |
| 2 | | | | | 1.0 | 10.5 | 16.0 | 2.0 | 2.0 | 5.5 |
| 21 | | | | | | 1.0 | 1.5 | 0.2 | 0.2 | 0.5 |
| 32 | | | | | | | 1.0 | 0.1 | 0.1 | 0.3 |
| 4 | | | | | | | | 1.0 | 1.0 | 2.8 |
| 4 | | | | | | | | | 1.0 | 2.8 |
| 11 | | | | | | | | | | 1.0 |

[FIG.11]

FP SIMILARITY EVALUATING PROCESS
START OF FP SIMILARITY EVALUATING PROCESS
READ TARGET FP
S201
ACQUIRE ALL RETENTION TIME POINTS (R1)
S202
LIST FILE NAMES OF ALL REFERENCE FPs
S203
n ← 1
S204
READ n-TH REFERENCE FP IN LIST (REFERENCE FPn)
S205
ACQUIRE ALL RETENTION TIME POINTS (R2)
S206
CALCULATE DEGREE OF MATCHING BETWEEN TARGET FP AND REFERENCE FPn FROM R1 AND R2 (RPn_min)
S207
SUBROUTINE 1 CALCULATION OF DEGREE OF MATCHING IN RETENTION TIME APPEARANCE PATTERN
PRESERVE RPn_min
S208
UPDATE n (n ← n + 1)
S209
HAVE ALL REFERENCE FP PROCESSES BEEN COMPLETED?
S210
NO
YES
SELECT REFERENCE FP WITH MINIMUM RPn_min
S211
END OF FP SIMILARITY EVALUATING PROCESS

[FIG.12]

SUBROUTINE 1 (CALCULATION OF DEGREE OF MATCHING BETWEEN RETENTION TIME APPEARANCE PATTERNS (RP))
START OF CALCULATING DEGREE OF MATCHING BETWEEN RETENTION TIME APPEARANCE PATTERNS
x ← R1, y ← R2
S1001
ACQUIRE NUMBER OF DATA PIECES OF "x" AND "y" (a, b)
S1002
i ← 1
S1003
ACQUIRE ALL DISTANCES FROM xi-TH RETENTION TIME POINT (f)
S1004
j ← 1
S1005
ACQUIRE ALL DISTANCES FROM yj-TH RETENTION TIME POINT (g)
S1006
ACQUIRE NUMBER OF DATA PIECES SATISFYING CONDITION OF "|INTER-RETENTION TIME POINT DISTANCE OF "f" - INTER-RETENTION TIME POINT DISTANCE OF "g"| < THRESHOLD VALUE" (m)
S1007
CALCULATE DEGREE OF MATCHING BETWEEN RETENTION TIME APPEARANCE PATTERNS OF "f" AND "g" (RPfg)
S1008
RPfg = (1 - (m / (a - b - m))) × (a - m - 1)
STORE RPfg (RP_all)
S1009
UPDATE "j" (j ← j + 1)
S1010
HAS PROCESS OF ALL RETENTION TIME POINTS OF "y" BEEN COMPLETED?
S1011
NO
YES
UPDATE "i" (i ← i + 1)
S1012
HAS PROCESS OF ALL RETENTION TIME POINTS OF "x" BEEN COMPLETED?
S1013
NO
YES
ACQUIRE MINIMUM VALUE FROM RP_all (RP_min)
S1014
END OF CALCULATING DEGREE OF MATCHING BETWEEN RETENTION TIME APPEARANCE PATTERNS

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20020028005 A1
- JP 2002214215 A


### Non-patent literature cited in the description


- *Pharmaceuticals monthly,* 1986, vol. 28 (3), 67-71
- General Rules for Preparations. Japanese Pharmacopoeia
- Industry Standard and Voluntarily Revision of ''Precautions. *148 Prescriptions for Medical Kampo Drug Formulation and in Guide to General Kampo Prescription,* 1978